Europäisches Patentamt

European Patent Office

Office européen des brevets

(11) Veröffentlichungsnummer: **0 289 003 B1**

(12) **EUROPÄISCHE PATENTSCHRIFT**

(45) Veröffentlichungstag der Patentschrift: **29.06.94**

(51) Int. Cl.5: **G01N 33/563**, //G01N33/537, G01N33/68,G01N33/74

(21) Anmeldenummer: **88106763.1**

(22) Anmeldetag: **27.04.88**

(54) Immunchemisches Verfahren und Reagenz zur Bestimmung eines polyvalenten Antigens in einer flüssigen Probe.

(30) Priorität: **28.04.87 DE 3714147**

(43) Veröffentlichungstag der Anmeldung:
**02.11.88 Patentblatt 88/44**

(45) Bekanntmachung des Hinweises auf die Patenterteilung:
**29.06.94 Patentblatt 94/26**

(84) Benannte Vertragsstaaten:
**AT BE CH DE ES FR GB GR IT LI LU NL SE**

(56) Entgegenhaltungen:
**EP-A- 0 074 271**
**EP-A- 0 105 714**
**EP-A- 0 147 848**
**EP-A- 0 205 198**

**BIOCHEMICAL JOURNAL, vol. 173, 1978, GB;
J. CARLSSON et al., Seiten 723-737&NUM;**

(73) Patentinhaber: **BOEHRINGER MANNHEIM GMBH**

**D-68298 Mannheim(DE)**

(72) Erfinder: **Stock, Werner, Dr.**
Grawolfstrasse 2
**D-8032 Gräfelfing(DE)**
Erfinder: **Baier, Manfred, Dr.**
**Tiefentalweg 10**
**D-8124 Seeshaupt(DE)**
Erfinder: **Kaspar, Klaus Peter, Dr.**
**Calle Bertoni y Cabrera**
**Barrio Luis A. de Herrara, Asuncion(PY)**
Erfinder: **Kirch, Peter, Dr.**
**Waisenhausstrasse 16**
**D-8120 Weilheim(DE)**

(74) Vertreter: **Huber, Bernhard, Dipl.-Chem. et al**
**Patentanwälte**
**H. Weickmann, Dr. K. Fincke**
**F.A. Weickmann, B. Huber**
**Dr. H. Liska, Dr. J. Prechtel, Dr. B. Böhm**
**Postfach 86 08**
**20**
**D-81635 München (DE)**

**Beschreibung**

Die Erfindung betrifft ein immunchemisches Verfahren und Reagenz zur Bestimmung eines polyvalenten, d. h. mindestens bifunktionellen Antigens in einer flüssigen Probe.

Die empfindliche Bestimmung von polyvalenten Antigenen (Peptide, Proteine) unter Verwendung von zwei Antikörpern, die gegen unterschiedliche Antigendeterminanten gerichtet sind, ist bekannt, z. B. aus J. Clin. Chem. Clin. Biochem. 18 (1980) 197-208. Zahlreiche Varianten dieses Verfahrens sind bekannt geworden, beispielsweise aus der DE-OS 34 00 027. Nach dieser OS wird zur Bestimmung des polyvalenten Antigens eine Inkubation mit drei verschiedenen Rezeptoren durchgeführt, von denen der erste und der dritte in flüssiger Phase gelöst vorliegen und mit dem Antigen bindefähig sind, während der zweite Rezeptor in fester Phase vorliegt und mit dem ersten Rezeptor bindefähig ist und der dritte Rezeptor markiert ist und mit dem ersten und zweiten Rezeptor nicht kreuzreagiert. Durch die auf verschiedene Weise durchführbare Inkubation des zu bestimmenden Antigens mit den drei Rezeptoren erhält man schließlich eine an die feste Phase gebundene Sandwich-Struktur, bei der der an die feste Phase gebundene zweite Rezeptor den ersten Rezeptor bindet, letzterer wieder das Antigen bindet und das Antigen schließlich den markierten dritten Rezeptor bindet.

EP-A-0 105 714 beschreibt ein Konjugat aus 2 miteinander verbundenen Antikörpern oder -fragmenten, wobei einer gegen den festphasengebundenen Reagenzpartner und der andere gegen das zu bestimmende Antigen gerichtet ist.

Ein Nachteil der auf diesem Prinzip basierenden Verfahren und Reagenzien liegt in einer nicht befriedigenden Stabilität der hierfür verwendeten Reagenzien. Dabei wird hier unter Stabilität die Konstanz der Eichkurve (Signal/Konzentration des Analyten) über einen längeren Zeitraum verstanden. Die unbefriedigende Stabilität hat zur Folge, daß die Eichkurven der frisch hergestellten Reagenzien nicht mit den Eichkurven des gelagerten Reagenz übereinstimmen. In der Regel beobachtet man, daß mit zunehmender Lagerung die Eichkurve flacher wird. Die Gründe hierfür sind nicht bekannt.

Aufgabe der Erfindung ist es nun, ein Verfahren und ein Reagenz der oben beschriebenen Art so zu verbessern, daß die Stabilität der Eichkurve über einen längeren Zeitraum als bisher gegeben ist, mit anderen Worten, die Zuverlässigkeit von Doppelantikörper-Sandwichverfahren (DASP) verbessert wird.

Erfindungsgemäß wird diese Aufgabe gelöst durch ein Verfahren zur quantitativen Bestimmung eines polyvalenten Antigens durch Inkubation mit drei verschiedenen Rezeptoren, von denen der erste (R1) und der dritte (R3) in flüssiger Phase gelöst vorliegen und mit dem Antigen bindefähig sind, der zweite Rezeptor (R2) in fester Phase vorliegt und mit (R1) bindefähig ist und (R3) eine Markierung trägt und mit (R1) und (R2) nicht kreuzreagiert, Trennung der festen von der flüssigen Phase und Messung der Markierung in einer der Phasen, welches dadurch gekennzeichnet ist, daß man einen ersten Rezeptor (R1) in Form eines Oligomeren oder Polymeren verwendet, der aus mindestens vier miteinander verbundenen Antikörpermolekülen oder Antikörpermolekül-Fragmenten besteht, die mit dem zu bestimmenden Antigen spezifisch binden.

Überraschend hat sich herausgestellt, daß man bei Verwendung eines oligomeren oder polymeren ersten Rezeptors (R1) eine wesentliche Verbesserung der Stabilität des Verfahrens bzw. des Reagenz erhält.

Als Rezeptoren werden im Rahmen der Erfindung spezifisch bindefähige Substanzen, insbesondere entweder spezifisch bindefähige komplette Antikörper, die polyklonal oder monoklonal sein können, deren Antikörperfragmente oder Konjugate von Antikörpern oder Antikörperfragmenten mit Haptenen oder Antigenen verwendet. Bevorzugt werden monoklonale Antikörper eingesetzt.

In den folgenden Erläuterungen werden unter der Bezeichnung "Antikörper" bzw. "Antikörpermoleküle" nicht nur die kompletten Antikörper, sondern auch deren bindungsfähige Fragmente, die dem Fachmann bekannt sind, verstanden. Als polyvalentes Antigen wird hier ein Antigen mit mehreren antigenen Determinanten verstanden.

Die beiden Rezeptoren R1 und R2 bilden ein miteinander spezifisch bindefähiges Substanzpaar. So kann beispielsweise der Rezeptor R2 ein gegen den Rezeptor R1 gerichtetes Immunglobulin oder Protein A sein, wenn der Rezeptor R1 ein Antikörper ist. Weiterhin kann der Rezeptor R2 Avidin, Streptavidin oder Biotin sein, wenn der Rezeptor R1 ein biotinylierter oder mit Avidin versehener Antikörper ist. Weitere Substanzpaare sind geeignet und dem Fachmann bekannt.

Der erste Rezeptor (R1) besteht aus miteinander vernetzten gleichen Antikörpern oder bindefähigen Fragmenten derselben, die monoklonal oder polyklonal sein können. Die einzelnen Antikörpermoleküle sind dabei durch bivalente oder polyvalente Linker (Brückenbildner) miteinander verbunden. Bevorzugt wird die Vernetzung durch bivalente Linker, da sie eine leichtere Steuerung des "Polymerisationsgrades" des Rezeptors ermöglichen. Die erfindungsgemäße Wirkung wird jedoch auch mit polyvalenten Linkern erzielt.

Damit durch die Linker die Bindungsstellen der einzelnen Antikörper, aus denen der Rezeptor (R1) besteht, nicht blockiert werden, sollten zweckmäßig nicht mehr als 10 Linker je Antikörpermolekül gebunden werden. Bevorzugt werden 2 bis 5 Linker je Antikörpermolekül. Je nach der Reaktivität der bindenden Funktionen des Linkers erhält man diese bevorzugten Kupplungsprodukte, wenn man Linker und Antikörper in einem molaren Verhältnis von etwa 1:1 bis 50:1 für die Vernetzung einsetzt.

Als Linker (Brückenbildner) können solche Verbindungen verwendet werden, welche reaktionsfähige Gruppen aufweisen, die in wäßriger Lösung mit den funktionellen Gruppen von Proteinen unter Ausbildung einer kovalenten Bindung zu reagieren vermögen. Dem Fachmann ist eine große Anzahl hierfür geeigneter bifunktioneller oder polyfunktioneller Linker bekannt. Typische Beispiele für im Rahmen der Erfindung gut geeignete homo- oder heterobifunktionelle und trifunktionelle Linker sind in der nachstehenden Tabelle 1 aufgeführt.

## T a b e l l e   1

| Kurzbe-<br>zeichnung | Chemische Bezeichnung |
|---|---|
| SPDP | N-succinimidyl 3-(2-pyridyldithio)-propionat |
| EADB | Ethyl 4-azidophenyl-1,4-dithiobutyrimidat ˙ HCl |
| FNPA | 4-Fluoro-3-nitrophenylazid |
| HSAB | N-Hydroxysuccinimidyl-4-azidobenzoat |
| MABI | Methyl-4-azidobenzoimidat ˙ HCl |
| MBS | m-Maleimidobenzoyl-N-hydroxysuccinimid-ester |
| NHS-ASA | N-Hydroxysuccinimidyl-4-azidosalicylsäure |
| MHS | m-Maleimidohexanoyl-N-hydroxysuccinimid-ester |
| PNP-DTP | p-Nitrophenyl-2-diazo-3,3,3-trifluoropropionat |
| SADP | N-Succinimidyl(4-azidophenyl)1,3'-dithiopropionat |
| SAND | Sulfosuccinimidyl-2-(m-azido-o-nitrobenzamido)-ethyl-1,3'-dithiopropionat |
| SANPAH | N-succinimidyl-6(4'-azido-2'-nitrophenyl-amino)hexanoat |
| SASD | Sulfosuccinimidyl 2-(p-azidosalicylamido)ethyl-1,3'-dithiopropionat |
| SIAB | N-Succinimidyl(4-iodoacetyl)aminobenzoat |
| SMCC | Succinimidyl-4-(N-maleinimidoethyl)cyclohexan-1-carboxylat |
| SMPB | Succinimidyl-4-(p-maleimidophenyl)butyrat |
| DSS | Disuccinimidylsuberat |
| DMS | Dimethylsuberimidat |
| Traut's Reagenz | 2-Iminothiolan |
| --- | 2,4,6 Trichlor-s-triazin |

Zur Durchführung der Vernetzung kann eine Lösung der miteinander zu verbindenden Antikörper mit den Linkermolekülen versetzt werden unter Bedingungen, die direkt zur Vernetzung führen. Das Ausmaß der Vernetzung wird in diesem Falle durch die Menge an zugegebenem Linker gesteuert.

Bevorzugt erfolgt die Vernetzung derart, daß die eine Hälfte der miteinander zu verbindenden Antikörper mit einem ersten Linker unter Bedingungen umgesetzt wird, bei der nur eine der Funktionen des Linkers zur Ausbildung einer Bindung mit dem Antikörper führt. In analoger Weise wird die andere Hälfte der zu vernetzenden Antikörper mit einem zweiten Linker umgesetzt. Die so erhaltenen beiden Antikörperderivate werden dann miteinander umgesetzt. Hierbei wird vorzugsweise ein molares Verhältnis der miteinander zu verbindenden Antikörperderivate zwischen 2:1 und 1:2 angewendet. Besonders bevorzugt ist ein Verhältnis von etwa 1:1.

Der Rezeptor (R1) enthält mindestens 4 Antikörpermoleküle, vorzugsweise 4 bis 20 Antikörpermoleküle, besonders bevorzugt 4 bis 12 Antikörpermoleküle.

Außer dem oben schon erläuterten Fall des aus einer einzigen Antikörperart zusammengesetzten Rezeptors (R1) (homologer, als Oligomer bzw. Polymer vorliegender Antikörper) kann der Rezeptor (R1) auch aus zwei verschiedenen Antikörpern zusammengesetzt werden, die gegenüber dem zu bestimmenden Antigen und gegenüber dem Rezeptor (R2) jeweils für sich eine gut spezifische Bindefähigkeit aufweisen. In diesem Falle erfolgt die Herstellung des kompletten Rezeptors (R1) durch die oben beschriebene bevorzugte Methode der individuellen Derivatisierung der beiden verschiedenen Antikörper und anschließende Umsetzung der beiden Derivate miteinander.

Für die Verwendung im Rahmen der Erfindung wird ein Rezeptor R1 bevorzugt, der hinsichtlich seines Polymerisationsgrades bzw. der Zahl der darin enthaltenen einzelnen Antikörpermoleküle eine möglichst gleichmäßige Zusammensetzung aufweist. Dies läßt sich auf einfache Weise durch chromatographische Vorreinigung erzielen.

Als Rezeptor (R2) wird ein an eine unlösliche Phase gebundener Rezeptor verwendet, der mit dem Rezeptor (R1) bindefähig ist. Vorzugsweise ist (R2) ein Antikörper im Sinne der obigen Definition. (R2) ist entweder mit einem Teil des aus homologen Antikörpern zusammengesetzten Rezeptors (R1) bindefähig oder bei einem heterogen zusammengesetzten Rezeptor (R1) mit derjenigen Komponente bindefähig, die zum zu bestimmenden Antigen die geringere Affinität aufweist. Vorzugsweise ist der Rezeptor (R2) gegen den Fc-Teil eines Antikörpermoleküls im Rezeptor (R1) gerichtet. Geeignet ist auch ein Rezeptor (R2), der allgemein gegen den Fc-Teil von Immunoglobulin gerichtet ist.

Der dritte Rezeptor (R3) ist ebenso wie der Rezeptor (R1) mit dem zu bestimmenden polyvalenten Antigen bindefähig. Er kann einen polyklonalen oder monoklonalen Antikörper enthalten. Handelt es sich um einen monoklonalen Antikörper, so wird er so gewählt, daß er sich an ein anderes Epitop wie (R1) bindet. Dies läßt sich auf einfache Weise erreichen, indem sowohl in (R1) als auch in (R3) jeweils ein monoklonaler Antikörper, der mit dem zu bestimmenden polyvalenten Antigen bindefähig ist, verwendet wird, wobei diese monoklonalen Antikörper verschiedene antigene Determinanten erkennen. Dies hat den Vorteil, daß beide Antikörper aus dem gleichen Versuchstier stammen können.

Der Rezeptor R(3) trägt außerdem noch eine Markierung. Die Markierung kann ein Enzym, eine radioaktive Substanz, ein Isotop, eine fluoreszierende oder chemilumineszierende Substanz sein, deren Bestimmung nach einer der dem Fachmann wohlbekannten Methoden durchgeführt wird. So kann beispielsweise, wenn die Markierung mit einem Enzym wie Peroxidase oder $\beta$-Galactosidase erfolgt, die Aktivität dieses Enzyms in der hierfür allgemein bekannten Weise gemessen werden, indem man ein bekanntes Nachweisreagenz für dieses Markierungsenzym, z. B. ein Farbreagenz, zusetzt und entweder in Anwesenheit der festen Phase oder nach Trennung mißt. Die gemessene Enzymaktivität ist dann ein Maß für die Menge an zu bestimmendem polyvalenten Antigen.

Ein weiterer Gegenstand der Erfindung ist ein Reagenz zur quantitativen Bestimmung eines polyvalenten Antigens enthaltend 3 Rezeptoren (R1, R2, R3), von denen (R1) und (R3) löslich und mit dem Antigen bindefähig sind, (R3) markiert ist und mit (R1) und (R2) nicht kreuzreagiert, welches dadurch gekennzeichnet ist, daß es einen ersten Rezeptor (R1) in Form eines Oligomeren oder Polymeren enthält, welches aus mindestens 4 miteinander Verbundenen Antikörpermolekülen oder Antikörpermolekülfragmenten besteht, die mit dem zu bestimmenden Antigen spezifisch binden.

Die bevorzugten Ausführungsformen dieses Reagenz sind vorstehend schon in Verbindung mit dem Bestimmungsverfahren eingehend erläutert worden und gelten analogerweise für die Zusammensetzung des Reagenz.

Durch die Erfindung wird eine Verbesserung der Stabilität der Reagenzien für heterogene Immunoassays, wie das Doppel-Antikörper-Sandwich-Verfahren erreicht, insbesondere hinsichtlich der Konstanz der Eichkurve über einen längeren Zeitraum. Durch die erzielte verbesserte Konstanz der Eichkurve wird die Zuverlässigkeit der Bestimmung erhöht.

Die folgenden Beispiele und Abbildungen erläutern dies weiter.

Fig. 1      zeigt ein Rotoreinsatzelement gemäß DE-AS 34 25 008.2

Fig. 2      zeigt eine LH-Eichkurve mit Rezeptor R1' (nicht vernetzt)

Fig. 3      zeigt eine LH-Eichkurve mit Rezeptor R1 (vernetzt)

4

**Beispiel 1**

Bestimmung von AFP

1. Herstellung der Reagenzien

1.1 Puffer I:
50 mmol/l Kalium-Phosphat-Puffer, pH 6,0, hergestellt durch Mischung von 50 mmol/l $K_2HPO_4$-Lösung und 50 mmol/l $KH_2PO_4$-Lösung bis zum Erreichen des pH-Wertes 6,0.

1.2 Puffer II:
Puffer II wird wie Puffer I hergestellt mit dem Unterschied, daß als pH-Wert der pH 7,5 eingestellt wird und daß der Puffer zusätzlich 10 g/l Rinderserumalbumin und 150 mmol/l NaCl enthält.

1.3 Rezeptor (R1′) (unvernetzter anti-AFP-Antikörper)
Als Rezeptor (R1′) wird ein monoklonaler Maus- anti-AFP-Antikörper der Subklasse $IgG_{2a}$ (ECACC 87041002) eingesetzt. Die diesen Antikörper enthaltende Ascitesflüssigkeit wird ad 1,8 M mit Ammoniumsulfat versetzt. Das Präzipitat wird in einem Puffer aus 15 mM Natriumphosphat, pH 7,0 und 50 mM Natriumchlorid aufgenommen. Die so erhaltene Lösung wird einer Passage über DEAE-Cellulose unterworfen. Das so erhaltene, mit AFP bindefähigen Antikörper enthaltende Eluat wird anschließend gefriergetrocknet.

1.4 Rezeptor (R1) (vernetzter anti-AFP-Antikörper)
1.4.1 Derivatisierung des AFP-spezifischen Antikörpers
10 mg Rezeptor (R1′) (monoklonaler Maus anti-AFP-Antikörper, s. 1.3) werden in 1 ml 0,1 M Natrium-Phosphat-Puffer pH 7,5 gelöst. Nach Zugabe von 0,1 mg Succinimidyl-3-(2-pyridyldithio)-propionat (SPDP) erfolgt eine Stunde Reaktion bei 25°C. Nach Umstellung des pH-Wertes mit Essigsäure auf pH 4,5 und Zugabe von 8 mg Dithiothreit erfolgt für weitere 20 Minuten Reaktion bei 25°C. Das Produkt wird mittels Passage über 10 ml ACA 202-Gel (Hersteller: LKB, Schweden) in einer Chromatographie-Säule in 10 mM Kalium-Phosphatpuffer pH 6,15 mit 25 mM NaCl und 10 mM $MgCl_2$ entsalzen. Das Produkt wird mittels eines handelsüblichen Ultrafiltrationsgerätes (Amicon Corp., USA) auf eine Protein-Konzentration von 14 mg/ml aufkonzentriert.

1.4.2 Derivatisierung des Vernetzungspartners
10 mg eines monoklonalen Maus-anti-hCG-Antikörpers der Subklasse $IgG_1$ (ECACC 87041001) (Herstellung wie unter 1.3 beschrieben) werden nach bekanntem Verfahren (Ishikawa et al., J. Immunoassay 4, (1983), S. 209 ff) mit N-succinimidyl-4(N-maleinimidomethyl)-cyclohexan-1-carboxylat (SMCC) derivatisiert. Entsalzung und Aufkonzentrierung erfolgt wie unter 1.4.1 beschrieben.

1.4.3 Konjugation
Je 10 mg der Antikörper-Derivate aus 1.4.1 und 1.4.2 werden zusammen bei pH 7,0 und 25°C für 1,5 Stunden zur Reaktion gebracht. Die Reaktion wird durch Zugabe von Cystein ad 1 mM (30 Minuten, 25°C) und anschließend Jodacetamid (1 Stunde, 25°C) gestoppt. Das Produkt wird wie unter 1.4.1 beschrieben entsalzen und aufkonzentriert.

1.5 Markierte Rezeptor (R3)-Lösung:
Als Rezeptor (R3) wird ein polyklonaler Maus-Anti-AFP-Antikörper eingesetzt wird, der nach einer immunsorptiven Reinigung, wie unter 1.3 angegeben, weiterbehandelt wird. Der vollständige Antikörper wird in bekannter Weise nach der Methode von R.R. Porter, Biochem. J. 73, (1959) S. 119, zum Fab-Fragment gespalten. Die erhaltenen Fab-Fragmente werden gemäß Ishikawa et al, J. of Immunoassay, 4 (1983) S. 209-327, mit β-Galactosidase gekoppelt. Die Rezeptor (R3)-Lösung wird in Puffer II auf eine Konzentration von 500 mU/ml (gemessen mit o-Nitrophenyl-β-Galactosid bei 37°) verdünnt.

1.6 Rezeptor (R2)-Lösung:
Schaf-anti-Maus-Fc$\gamma$-Antiserum wird ad 1,8 M mit Ammoniumsulfat versetzt. Das Präzipitat wird in einem Puffer aus 15 mM Natriumphosphat, pH 7,0 und 50 mM Natriumchlorid aufgenommen. Die so erhaltene Lösung wird einer Passage über DEAE-Zellulose unterworfen. Das den spezifischen Antikörper enthaltende Eluat wird in Puffer I auf eine Proteinkonzentration von 50 μg/ml verdünnt.

1.7 Substratlösung:

| Chlorphenolrot-$\beta$-galactosid (hergestellt nach DE 33 45 748) | 5 mmol/l (3,05 g/l) |
|---|---|
| HEPES | 70 mmol/l (16,7 g/l) |
| NaCl | 154 mmol/l (9 g/l) |
| Rinderserumalbumin | 0,3 % ( 3 g/l) |
| Tween® 20 | 0,2 % (2 g/l) |
| pH (mit NaOH) | 7,25 |

2. Herstellung von Reagenzträgern

2.1 Reagenzträger 1' (Vergleich; mit unvernetztem Rezeptor (R1')) 40 $\mu$l einer Lösung, die pro Liter 100 mmol Natriumphosphat pH 7,3 (37°C), 2 mmol Magnesiumchlorid, 9 g Natriumchlorid, 5 g Rinderserumalbumin, 5 mg Anti-AFP-monoklonale Antikörper aus Maus (Rezeptor R1') (200 ng AK/Test), 1.000 U Anti-AFP-Antikörper-(Maus)-Fab-Fragment-$\beta$-Galactosidase-Konjugat (Rezeptor-R3-Lösung, Aktivität bestimmt mit ortho-Nitrophenyl-$\beta$-D-galactosid bei 37°C) enthält, wird auf ein Vlies aufgetropft, das aus kommerziellem Polyesterpapier besteht. Anschließend wird bei Raumtemperatur getrocknet. Diese Vliese werden bis zu ihrer Verwendung bei 4°C und einer relativen Luftfeuchtigkeit von 20 % aufbewahrt.

2.2 Reagenzträger 1 (erfindungsgemäß; mit vernetztem Rezeptor (R1)): Die Herstellung erfolgt wie für Reagenzträger 1', bis auf den Unterschied, daß anstelle des unvernetzten monoklonalen anti-AFP-Antikörpers (Rezeptor (R1')) ein vernetzter monoklonaler anti-AFP-Antikörper aus Maus (Rezeptor (R1)) benutzt wird.

2.3 Reagenzträger 2:

Auf ein Zellulose-Vlies werden nach dem Bromcyan-Aktivierungsverfahren (DE-OS 17 68 512) Schaf-Antikörper gegen den Fc$\gamma$-Teil von Maus-Antikörpern (Rezeptor-R3-Lösung) fixiert, wobei pro g Fasermaterial 10 $\mu$g Antikörper zur Fixierung angeboten werden. Durch Waschen wird ungekoppelter Antikörper entfernt und das Vlies schonend bei Raumtemperatur getrocknet. Die Lagerung der so erhaltenen Vliese folgt analog Reagenzträger 1.

3. Durchführung der Bestimmung

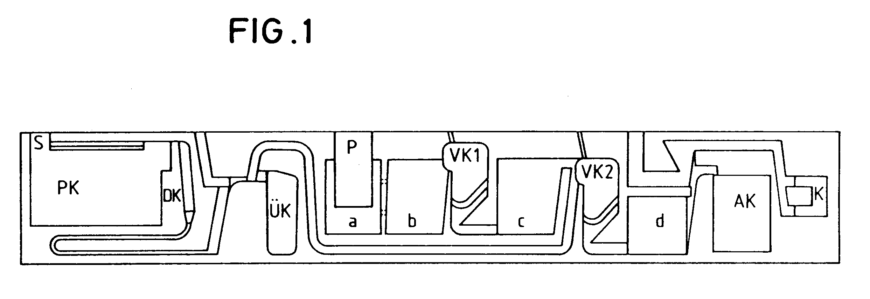

Die Bestimmung mit Hilfe dieser beiden Reagenzträger 1 und 2, bzw. 1' und 2, erfolgt mit der in der DE-AS 34 25 008.5 beschriebenen Vorrichtung zur Durchführung analytischer Bestimmungen (Fig. 1).

Diese lehrt ein Rotoreinsatzelement für Zentrifugalanalysenautomaten, bestehend aus einem Formkörper, der eine Probenauftragskammer, die mit einer Mehrzahl von Reagenzfeldern in Verbindung steht, die jeweils ein mit einem bestimmten Reagenz imprägniertes saugfähiges Trägermaterial enthalten, wenigstens eine Mischventilkammer und eine Meßkammer aufweist, die zusammen einen Probenflüssigkeitstransportweg bilden, der von radial innen nach radial außen führt, wenn das Einsatzelement auf dem Rotor befestigt ist und weiter wenigstens eine weitere Kammer für die Aufnahme einer Flüssigkeit und einen Transportweg, der von dieser Kammer zur Meßführung führt und mit dem Probenflüssigkeitstransportweg mindestens teilweise identisch ist, aufweist. Dabei führt der Probenflüssigkeitstransportweg von einer Probenauftragskammer (P) über eine mit saugfähigem Material gefüllte, Puffer enthaltende Kammer (a), eine Kammer (c) und eine zwischen den Kammern (a) und (c) angeordnete erste Ventilkammer (Vk1) zu einer zweiten Ventilkammer (Vk2) und von dieser über die Kammer (d) und über eine Auffangkammer (AK) zur Meßkammer (K). Zur Aufnahme einer weiteren Flüssigkeit ist eine als Pumpenkammer ausgebildete Substratkammer (PK) vorgesehen, welche über eine aus Dosierkammer (DK) und Kapillare (Kap) bestehende Dosiereinrichtung und eine Überlaufkammer (ÜK) mit der zweiten Ventilkammer (Vk2) verbunden ist. Fig. 1 zeigt schematisch das verwendete Rotoreinsatzelement.

Reagenzträger 1 bzw. 1' wird auf Feld c des Rotoreinsatzelements plaziert und Reagenzträger 2 auf Feld d. Dabei werden 40 $\mu$l einer mit 0,9% NaCl-Lösung 1:3 verdünnten Probe durch eine Öffnung am oberen Rand direkt auf das Feld a pipettiert. 270 $\mu$l Substratlösung werden in Kammer PK pipettiert. Durch ein geeignetes Zentrifugations-Programm, bei dem hohe Drehzahlen mit Stillstand abwechseln, werden dann Probe und Substratlösung in Richtung Trennmatrix und Küvette gefördert.

Dabei werden im Verlauf des Programms die Rezeptoren (R3) und (R1) bzw. (R1') durch die Probenflüssigkeit vom Feld c eluiert und die homogene Mischung anschliessend zur Reaktion gebracht. Auf Feld d werden die gebildeten Komplexe an den Rezeptor (R2) gebunden. Der Transfer der Probe von Feld c nach d erfolgt innerhalb sehr kurzer Zeit.

Die Substratlösung wird durch die Dosierkammer DK in Portionen geteilt, von denen die ersten zum Auswaschen von überschüssigem, nicht komplexiertem Konjugat dienen. Die über Komplexbildung auf d gebundene $\beta$-Galactosidase-Aktivität ist proportional zur in der Probe enthaltenen AFP-Menge bzw. zum Probenleerwert. Diese Aktivität wird mit einer weiteren Substratportion bestimmt, wobei das Substrat in einer fünfmünütigen Reaktion zu farbigen Produkten umgesetzt wird. Die gebildete Farbe und die weitere Farbentwicklung/min in der flüssigen Phase werden in der Küvette bei 576 nm gemessen.

Unter diesen Bedingungen wurden Eichgeraden sowohl mit unbelasteten als auch mit 3 Wochen bei 35°C belasteten Rotoreinsatzelementen einschließlich ihrer Reagenzträger ermittelt. Dabei wurden folgende Resultate erhalten:

| | Ergebnisse in Extinktionseinheiten bei Verwendung von | | | |
| | unvernetztem Rezeptor R1' | | vernetztem Rezeptor R1 | |
| AFP-Konzentration (IU/ml) | 0 | 70 | 0 | 70 |
|---|---|---|---|---|
| a) Einsatzelement mit Reagenzträgern ohne Temperaturbelastung | 0,303 | 4,955 | 0,327 | 5,702 |
| b) Einsatzelement mit Reagenzträgern 3 Wochen 35°C belastet | 0,302 | 3,652 | 0,301 | 4,970 |
| c) Eichkurvenstabilität[1] nach Belastung) | | 72% | | 87% |

[1] Berechnung der Eichkurvenstabilität:

$$\frac{\text{Signaldifferenz (0/70 IU/ml AFP) Einsatzelement belastet (b)}}{\text{Signaldifferenz (0/70 IU/ml AFP) Einsatzelement unbelastet (a)}} \times 100$$

**Beispiel 2**

2.1 Herstellung der Reagentien

2.1.1 Rezeptor (R1') ( Vergleich; unvernetzter anti-hLH-Antikörper)
Als Rezeptor (R1') wird ein monoklonaler Maus-anti-hLH-Antikörper der Subklasse IgG$_1$ (NCACC 84122001) eingesetzt. Die diesen Antikörper enthaltende Ascitesflüssigkeit wird ad 1,8 M mit Ammoniumsulfat versetzt. Das Präzipitat wird in einem Puffer aus 15 mM Natriumphosphat, pH 7,0 und 50 mM Natriumchlorid aufgenommen. Die so erhaltene Lösung wird einer Passage über DEAE-Cellulose unterworfen. Das so erhaltene, mit hLH-bindefähigen Antikörper enthaltende Eluat, wird anschließend gefriergetrocknet.
2.1.2 Rezeptor (R1) (erfindungsgemäß; vernetzter anti-hLH-Antikörper)
Im Gegensatz zu Beispiel 1 wird im Beispiel 2 ein mit sich selbst vernetzter Antikörper verwendet. Zur Herstellung wird Rezeptor (R1') nach Beispiel 1, 1.4.1 und 1.4.2 derivatisiert.

7

Je 10 mg dieser Antikörper-Derivate werden zusammen bei pH 7,0 und 25°C für 1,5 Stunden zur Reaktion gebracht. Die Reaktion wird durch Zugabe von Cystein ad 1 mM (30 Minuten, 25°C) und anschliessend Jodacetamid (1 Stunde, 25°C) gestoppt. Das Produkt wird wie unter Beispiel 1, 1.4.1 beschrieben, entsalzen und aufkonzentriert.

2.1.3 Markierte Rezeptor (R3)-Lösung

(R3) ist ein Konjugat bestehend aus $\beta$-Galactosidase und einem monoklonalen Anti-LH-Antikörper-Fab-Fragment. Das Fab-Fragment wird in bekannter Weise, nach R.R. Porter, Biochem. J. 73 (1959), S. 119, durch Spaltung des vollständigen monoklonalen Anti-LH-Antikörpers (NCACC 84122005) gewonnen. Die Spezifität von (R3) ist von (R1) verschieden. (R3) erkennt auf dem LH-Molekül unterschiedliche antigene Determinanten und kann mit (R1) einen Sandwich bilden. Antikörperproduktion und Konjugatsynthese entsprechen Beispiel 1, Punkt 1.5.

2.1.4 Sonstige Reagentien

Die sonstigen Reagentien (z. B. Puffer I und II, Rezeptor (R2)-Lösung und Substratlösung) sind identisch mit den in Beispiel 1 verwendeten Reagentien.

2.2 Herstellung von Reagenzträgern

2.2.1 Reagenzträger 1' (mit unvernetztem Rezeptor (R1')) 40 $\mu$l einer Lösung, die pro Liter 100 mmol Natriumphosphat pH 7,3 (37°C), 2 mmol Magnesiumchlorid, 9 g Natriumchlorid, 5 g Rinderserumalbumin, 10 mg bzw. 20 mg Anti-hLH-monoklonale Antikörper aus Maus (Rezeptor R1') (400 ng AK/Test bzw. 800 ng AK/Test), 1.000 U Anti-hLH-Antikörper-(Maus)-Fab-Fragment-$\beta$-Galactosidase-Konjugat (Rezeptor R3-Lösung, Aktivität bestimmt mit ortho-Nitrophenyl-$\beta$-D-galactosid bei 37°C) enthält, wird auf ein Vlies aufgetropft, das aus kommerziellem Polyesterpapier besteht. Anschließend wird bei Raumtemperatur getrocknet. Diese Vliese werden bis zu ihrer Verwendung bei 4°C und einer relativen Luftfeuchtigkeit von 20% aufbewahrt.

2.2.2 Reagenzträger 1:

Die Herstellung erfolgt wie für Reagenzträger 1', bis auf den Unterschied, daß anstelle von Rezeptor (R1') ein mit sich selbst vernetzter Rezeptor (R1) benutzt wird.

2.2.3 Reagenzträger 2:

Auf ein Zellulose-Vlies werden nach dem Bromcyan-Aktivierungsverfahren (DE-OS 17 68 512) Schaf-Antikörper gegen den Fc$\gamma$-Teil von Maus-Antikörpern (Rezeptor-R2-Lösung) fixiert, wobei pro g Fasermaterial 10 $\mu$g Antikörper zur Fixierung angeboten werden. Durch Waschen wird ungekoppelter Antikörper entfernt und das Vlies schonend bei Raumtemperatur getrocknet. Die Lagerung der so erhaltenen Vliese folgt analog Reagenzträger 1.

3. Durchführung der Bestimmung:

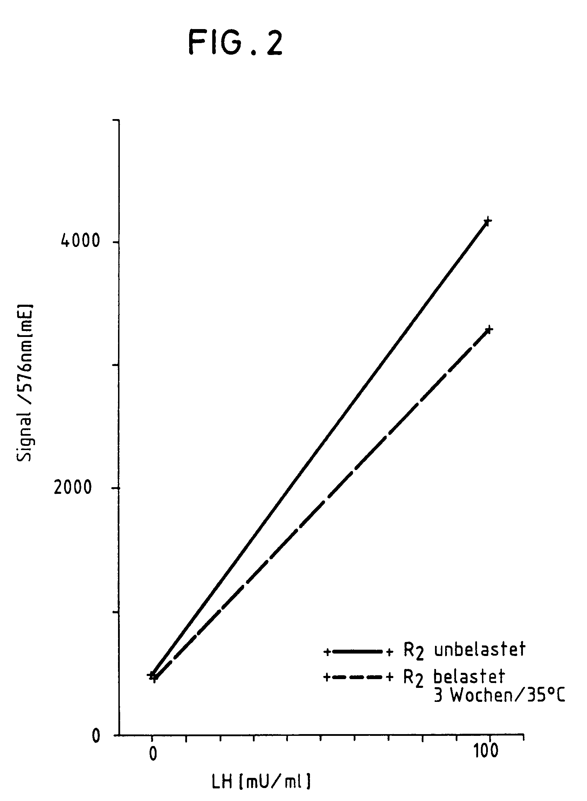

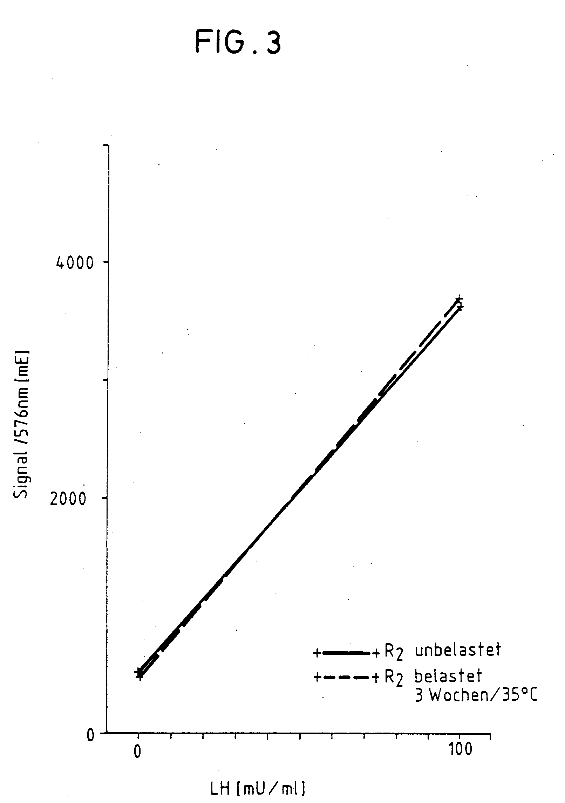

Die Durchführung der Bestimmung erfolgt analog Beispiel 1. Fig. 2 und Fig. 3 zeigen die Ergebnisse bei Verwendung von 400 ng/AK/Test im Reagenzträger 1' bzw. 1.

Tabelle II zeigt die Ergebnisse für 400 bzw. 800 ng AK/Test.

T a b e l l e    II

A Ergebnisse bei Verwendung von 400 ng Rezeptor R1' oder
  R1 im Reagenzträger 1' bzw. 1

|  | unvernetzter Rezeptor R1' | | vernetzter Rezeptor R1 | |
|  | LH-Konzentration (mU/ml) | | LH-Konzentration (mU/ml) | |
|  | 0 | 100 | 0 | 100 |
|---|---|---|---|---|
| Reagenzträger 2 unbelastet | 487* | 4177 | 510 | 3635 |
| Reagenzträger 2 3 Wochen 35°C belastet | 462 | 3288 | 473 | 3699 |
| Eichkurvenstabilität nach Belastung ** | | 77% | | 100% |

B Ergebnisse bei Verwendung von 800 ng Rezeptor R1' oder R1 im
  Reagensträger 1' bzw. 1

| Reagenzträger 2 unbelastet | 460 | 3430 | 481 | 4184 |
|---|---|---|---|---|
| Reagenzträger 2 3 Wochen 35°C belastet | 422 | 2637 | 508 | 4003 |
| Eichkurvenstabilität nach Belastung** | | 75% | | 94% |

* Alle Messungen wurden bei $\lambda$ = 576 nm bei einer Schichtdicke von 0,3 cm durchgeführt und auf Schichtdicke
  d = 1 cm umgerechnet.
  Angegeben ist der Mittelwert je 4 Bestimmungen in Milliextinktionen.

** Berechnung der Eichkurvenstabilität:

$$\frac{\text{Signaldifferenz (0/100 mU/ml LH) } \sigma \text{ belastet}}{\text{Signaldifferenz (o/100 mU/ml LH) unbelastet}} \times 100$$

**Patentansprüche**

1. Verfahren zur quantitativen Bestimmung eines polyvalenten Antigens durch Inkubation mit drei verschiedenen Rezeptoren, von denen der erste (R1) und der dritte (R3) in flüssiger Phase gelöst vorliegen und mit dem Antigen bindefähig sind, der zweite Rezeptor (R2) in fester Phase vorliegt und mit (R1) bindefähig ist und (R3) eine Markierung trägt und mit (R1) und (R2) nicht kreuzreagiert, Trennung der festen von der flüssigen Phase und Messung der Markierung in einer der Phasen, **dadurch gekennzeichnet,** daß man einen ersten Rezeptor (R1) in Form eines Oligomeren oder Polymeren verwendet, welches aus mindestens 4 miteinander verbundenen Antikörpermolekülen oder Antikörpermolekülfragmenten besteht, die mit dem zu bestimmenden Antigen spezifisch binden.

2. Verfahren nach Anspruch 1, **dadurch gekennzeichnet,** daß (R1) aus miteinander vernetzten gleichen monoklonalen oder polyklonalen Antikörpern oder bindefähigen Fragmenten derselben besteht.

3. Verfahren nach Anspruch 1, **dadurch gekennzeichnet,** daß (R1) aus miteinander vernetzten ungleichen mono- oder polyklonalen Antikörpern oder bindefähigen Fragmenten derselben besteht.

4. Verfahren nach einem der Ansprüche 1 bis 3, **dadurch gekennzeichnet,** daß die einzelnen Antikörperbestandteile von (R1) durch di- oder polyvalente Linker verbunden sind.

5. Verfahren nach Anspruch 4, **dadurch gekennzeichnet,** daß man einen Rezeptor (R1) verwendet, der 4 bis 12 Antikörpermoleküle oder Fragmente davon enthält.

6. Verfahren nach einem der vorhergehenden Ansprüche, **dadurch gekennzeichnet,** daß man (R1) in einer Konzentration einsetzt, die dem 0,5- bis 1fachen der Konzentration von (R2) entspricht.

7. Verfahren nach einem der vorhergehenden Ansprüche, **dadurch gekennzeichnet,** daß man bei aus gleichen kompletten Antikörpern bestehenden (R1) einen Rezeptor (R2) verwendet, der mit einem Teil von (R1) bindefähig ist.

8. Verfahren nach Anspruch 7, **dadurch gekennzeichnet,** daß (R2) mit dem Fc-Teil des Antikörpers in (R1) bindefähig ist.

9. Verfahren zur Herstellung eines Rezeptors (R1) für das Verfahren der Ansprüche 1 bis 8, **dadurch gekennzeichnet,** daß je 1/4 bis 3/4 der Menge an zu verbindenden Antikörpern oder bindefähigen Fragmenten davon mit einer Funktion je eines di- oder polyvalenten Linkers zu Derivaten umgesetzt und dann zwei dieser Derivate miteinander unter Bedingungen verknüpft werden, bei denen die zweite oder weitere Funktionen der Linker reagiert.

10. Verfahren nach Anspruch 9, **dadurch gekennzeichnet,** daß die beiden Derivate im molaren Verhältnis 2:1 bis 1:2 miteinander umgesetzt werden.

11. Reagenz zur quantitativen Bestimmung eines polyvalenten Antigens, enthaltend 3 Rezeptoren (R1, R2, R3), von denen (R1) und (R3) löslich und mit dem Antigen bindefähig sind, (R3) markiert ist und mit (R1) und (R2) nicht kreuzreagieren, **dadurch gekennzeichnet,** daß es einen ersten Rezeptor (R1) in Form eines Oligomeren oder Polymeren enthält, welches aus mindestens 4 miteinander verbundenen Antikörpermolekülen oder Antikörpermolekülfragmenten besteht, die mit dem zu bestimmenden Antigen spezifisch binden.

12. Reagenz nach Anspruch 11, **dadurch gekennzeichnet,** daß (R1) aus miteinander vernetzten, gleichen oder ungleichen, monoklonalen oder polyklonalen Antikörpern oder bindefähigen Fragmenten derselben besteht.

13. Reagenz nach Anspruch 12, **dadurch gekennzeichnet,** daß (R1) 4 bis 12 Antikörpermoleküle oder Fragmente davon enthält.

14. Reagenz nach einem der Ansprüche 11 bis 13, **dadurch gekennzeichnet,** daß (R1) in der 0,5- bis 1-fachen aktiven Menge von (R2) vorliegt.

## EP 0 289 003 B1

**Claims**

1. Process for the quantitative determination of a polyvalent antigen by incubation with three different receptors of which the first (R1) and the third (R3) are present dissolved in liquid phase and are bindable with the antigen, the second receptor (R2) is present in solid phase and is bindable with (R1) and (R3) carries a labelling and does not cross-react with (R1) and (R2), separation of the solid from the liquid phase and measurement of the labelling in one of the phases, characterised in that one uses a first receptor (R1) in the form of an oligomer or polymer which consists of at least 4 antibody molecules or antibody molecule fragments bound with one another, which bind specifically with the antigen to be determined.

2. Process according to claim 1, characterised in that (R1) consists of identical monoclonal or polyclonal antibodies or of bindable fragments thereof cross-linked with one another.

3. Process according to claim 1, characterised in that (R1) consists of different mono- or polyclonal antibodies or of bindable fragments thereof cross-linked with one another.

4. Process according to one of claims 1 to 3, characterised in that the individual antibody components of (R1) are bound by di- or polyvalent linkers.

5. Process according to claim 4, characterised in that one uses a receptor (R1) which contains 4 to 12 antibody molecules or fragments thereof.

6. Process according to one of the preceding claims, characterised in that one uses (R1) in a concentration which corresponds to 0.5 to 1 fold of the concentration of (R2).

7. Process according to one of the preceding claims, characterised in that, in the case of (R1) consisting of the same complete antibodies, one uses a receptor (R2) which is bindable with a part of (R1).

8. Process according to claim 7, characterised in that (R2) is bindable with the Fc part of the antibody in (R1).

9. Process for the preparation of a receptor (R1) for the process according to claims 1 to 8, characterised in that in each case 1/4 to 3/4 of the amount of antibody or bindable fragments thereof to be bound is reacted with, in each case, a function of a di- or polyvalent linker to give derivatives and then two of these derivatives are linked with one another under conditions under which the second or further functions of the linker react.

10. Process according to claim 9, characterised in that the two derivatives are reacted with one another in the molar ratio of 2:1 to 1:2.

11. Reagent for the quantitative determination of a polyvalent antigen containing three receptors (R1, R2, R3) of which (R1) and (R3) are soluble and bindable with the antigen, (R3) is labelled and does not cross-react with (R1) and (R2), characterised in that it contains a first receptor (R1) in the form of an oligomer or polymer which consists of at least 4 antibody molecules or antibody molecule fragments bound with one another, which bind specifically with the antigen to be determined.

12. Reagent according to claim 11, characterised in that (R1) consists of identical or different monoclonal or polyclonal antibodies or bindable fragments thereof cross-linked with one another.

13. Reagent according to claim 12, characterised in that (R1) contains 4 to 12 antibody molecules or fragments thereof.

14. Reagent according to one of claims 11 to 13, characterised in that (R1) is present in the 0.5 to 1 fold active amount of (R2).

11

EP 0 289 003 B1

**Revendications**

1. Procédé de détermination quantitative d'un antigène polyvalent par incubation avec trois récepteurs différents parmi lesquels le premier (R1) et le troisième (R3) sont présents dissous en phase liquide et sont capables de se lier à l'antigène, le second récepteur (R2) est présent en phase solide et est capable de se lier avec (R1) et (R3) porte un marqueur et ne présente pas de réaction croisée avec (R1) et (R2), séparation de la phase solide et de la phase liquide et mesure du marqueur dans l'une des phases, caractérisé en ce que l'on utilise un premier récepteur (R1) sous forme d'un oligomère ou d'un polymère qui consiste en au moins 4 molécules d'anticorps ou fragments de molécules d'anticorps liés entre eux, qui se lient spécifiquement à l'antigène à déterminer.

2. Procédé selon la revendication 1, caractérisé en ce que (R1) consiste en anticorps monoclonaux ou polyclonaux identiques réticulés entre eux ou en fragments de ces anticorps qui sont capables de se lier.

3. Procédé selon la revendication 1, caractérisé en ce que (R1) consiste en anticorps mono- ou polyclonaux différents réticulés entre eux ou en fragments de ces anticorps qui sont capables de se lier.

4. Procédé selon l'une des revendications 1 à 3, caractérisé en ce que les différents constituants d'anticorps de (R1) sont reliés par des lieurs di- ou polyvalents.

5. Procédé selon la revendication 4, caractérisé en ce que l'on utilise un récepteur (R1) qui contient 4 à 12 molécules ou fragments de molécules d'anticorps.

6. Procédé selon l'une des revendications précédentes, caractérisé en ce que l'on utilise (R1) en une concentration qui correspond à 0,5 à 1 fois la concentration de (R2).

7. Procédé selon l'une des revendications précédentes, caractérisé en ce que, lorsque (R1) consiste en anticorps complets identiques, l'on utilise un récepteur (R2) qui est capable de se lier à une partie de (R1).

8. Procédé selon la revendication 7, caractérisé en ce que (R2) est capable de se lier à la partie Fc de l'anticorps dans (R1).

9. Procédé de préparation d'un récepteur (R1) pour le procédé des revendications 1 à 8, caractérisé en ce que 1/4 à 3/4 de la quantité d'anticorps à lier ou de fragments de ces anticorps qui sont capables de se lier sont mis à réagir avec une fonction d'un lieur di- ou polyvalent pour former des dérivés puis deux de ces dérivés sont reliés entre eux dans des conditions dans lesquelles la seconde fonction ou d'autres fonctions des lieurs réagissent.

10. Procédé selon la revendication 9, caractérisé en ce que les deux dérivés sont mis à réagir entre eux dans le rapport molaire de 2:1 à 1:2.

11. Réactif pour la détermination quantitative d'un antigène polyvalent, contenant trois récepteurs (R1, R2, R3) parmi lesquels (R1) et (R3) sont solubles et sont capables de se lier à l'antigène, (R3) est marqué et ne présente pas de réaction croisée avec (R1) et (R2), caractérisé en ce qu'il contient un premier récepteur (R1) sous forme d'un oligomère ou d'un polymère qui consiste en au moins quatre molécules d'anticorps liées entre elles ou fragments de molécules d'anticorps liés entre eux qui se lient de manière spécifique à l'antigène à déterminer.

12. Réactif selon la revendication 11, caractérisé en ce que (R1) consiste en anticorps monoclonaux ou polyclonaux, identiques ou différents, réticulés entre eux ou en fragments de ces anticorps qui sont capables de se lier.

13. Réactif selon la revendication 12, caractérisé en ce que (R1) contient 4 à 12 molécules d'anticorps ou fragments de telles molécules.

12

**14.** Réactif selon l'une des revendications 11 à 13, caractérisé en ce que (R1) est présent à raison de 0,5 à 1 fois la quantité active de (R2).

FIG.1

EP 0 289 003 B1

# FIG.2

# FIG.3

Signal /576nm [mE]

4000

2000

0

0     100

LH [mU/ml]

+————+ R$_2$ unbelastet

+— — —+ R$_2$ belastet
3 Wochen/35°C